# DEMANDE DE BREVET EUROPEEN

(11) **EP 0 888 773 A1**
(43) Date de publication de la demande: **07.01.1999**
(21) Numéro de dépôt: 97111374.1
(22) Date de dépôt: 05.07.1997
(51) Int. Cl.: A61K 31/20, A61K 7/48

(54) **Utilisation de l'acide pétrosélinique pour le traitement des inflammations des tissus superficiels**

(71) Demandeur: SOCIETE DES PRODUITS NESTLE S.A., 1800 Vevey (CH)
(72) Inventeur: Malnoe, Armand, 1040 Dommartin (CH); Baur, Markus, 1004 Lausanne (CH); Fay, Laurent, 1073 Savigny (CH)

(57) **Abrégé**

Utilisation de l'acide pétrosélinique, natif ou estérifié, pour la préparation d'une composition destinée à activer la β-oxydation peroxisomale des acides gras dans les tissus superficiels d'un mammifère de sorte à pouvoir traiter ou prévenir les inflammations et/ou moduler le métabolisme des lipides des tissus superficiels.

## Description

L'invention se rapporte à une nouvelle utilisation d'une composition alimentaire, cosmétique et/ou pharmaceutique contenant de l'acide pétrosélinique.

La plupart des compositions cosmétiques et/ou dermatologiques contiennent, à une concentration plus ou moins élevée, une phase grasse généralement constituée d'au moins une huile, une matière grasse et/ou une cire, par exemple sous forme d'émulsions du type eau-dans-huile ou huile-dans-eau, de gels huileux, et de solutions huileuses.

Les huiles constituent généralement une proportion importante de cette phase grasse et peuvent être d'origines très variée. Il peut s'agir notamment d'huiles végétales, animales, minérales ou encore d'huiles synthétiques.

Parmi ces huiles, les huiles végétales constituent une classe de choix car la tendance actuelle est plus orientée vers l'utilisation de produits d'origine naturelle. Ces huiles végétales sont généralement constituées de triglycérides d'acides gras.

FR9412006 décrit à cet effet une composition cosmétique ou dermatologique destinée à hydrater la peau sèche, contenant une huile végétale riche en acide pétrosélinique extraite de graines d'ombellifères.

EP116439 divulgue que certains acides gras, dont l'acide pétrosélinique, ont une forte activité d'inhibition de la 5α-réductase et peuvent être utilisés de ce fait dans les produits toniques pour le traitement des cheveux.

Par ailleurs, Keller *et al*. expliquent que certains acides gras, dont l'acide pétrosélinique, peuvent avoir potentiellement un effet modulateur sur le métabolisme des lipides dans les cellules de foie lorsqu'ils activent les récepteurs induisant la prolifération des péroxisomes impliqués dans la dégradation des acides gras par β-oxydation (Proc. Natl. Acad. Sci. USA, 90, 2160-2164, 1993).

En outre, on sait également que, dans les cellules du foie, les activateurs de péroxysomes peuvent également augmenter la dégradation du leukotriène B4, et donc contrôler la durée d'une réponse inflammatoire (Devchand *et al*., Nature, 384, 39-43, 1996).

A ce jour, le métabolisme de l'acide pétrosélinique dans les tissus superficiels n'est pas connu, et notamment son incorporation dans les phospholipides sous forme native ou sous forme de produit de β-oxydation. On ne connaît donc pas son effet anti-inflammatoire et/ou modulateur du métabolisme des lipides dans les tissus superficiels.

La présente invention se rapporte ainsi à l'utilisation de l'acide pétrosélinique pour la préparation d'une composition destinée à activer la β-oxydation péroxisomale des acides gras dans les tissus superficiels d'un mammifère de sorte à pouvoir traiter ou prévenir les inflammations et/ou moduler le métabolisme des lipides dans les tissus superficiels.

Les tissus superficiels d'un mammifère peuvent être les cellules constituant la peau, le cuir chevelu, l'oeil, ou les muqueuses orale, buccale, nasale ou vaginale, par exemple.

Par "modulation du métabolisme des lipides" on entend plus particulièrement le catabolisme des médiateurs lipidiques liés à l'inflammation, la différentiation, la prolifération et/ou la fonction barrière des tissus superficiels.

De même, l'inflammation des tissus superficiels doit être comprise comme le phénomène physiologique impliquant la production de cytokines pro-inflammatoires, comme le TNFα, par les cellules des tissus superficiels, par exemple les kératinocytes et les cellules épithéliales de comée, et les cellules du système immunitaire contenues dans ces tissus (lymphocytes, cellules de Langerhans, ect...). L'inflammation peut résulter d'une infection, d'une allergie, d'une plaie, d'une maladie auto-immune, et d'une exposition à des radiations et/ou des agents irritants et/ou des agents sensibilisants, par exemple.

L'acide pétrosélinique peut ainsi être utilisé en particulier pour le traitement ou la prophylaxie de maladies de la peau ou du cuir chevelu, notamment contre les inflammations liées au psoriasis, l'érythème (coup de soleil), l'eczéma, la dermatite seborrheique, l'*alopecia areata*, la mycose, l'acné ou autres dermatoses, par exemple. Les applications peuvent aussi être étendues aux inflammations de l'oeil (inflammation de la cornée) et des muqueuses, notamment des muqueuses orale, nasale, buccale et vaginale.

La composition peut être appliquée directement sur les tissus superficiels de sorte que l'acide pétrosélinique puisse diffuser à travers les cellules et y être assimilé. Cette composition peut aussi être injectée sous les tissus superficiels, par exemple au moyen d'une injection sous-cutanée. Dans les deux cas, on considère que l'application de l'acide pétrosélinique est topique, c'est à dire, appliquée directement sur, ou sous, les tissus superficiels.

Cette composition peut aussi être administrée oralement de sorte que l'acide pétrosélinique puisse agir sur les muqueuses (les muqueuses buccale, de l'oesophage, stomacale et intestinale), et/ou qu'il puisse passer dans la circulation sanguine et être apporté directement aux cellules de la peau, de l'oeil ou des muqueuses, par exemple.

Cette composition peut aussi être appliquée dans les voie nasale, au moyen d'un diffuseur, d'un gel et/ou encore d'un liquide physiologique destiné classiquement au lavement des voies nasales.

L'acide pétrosélinique, encore appelé l'acide δ-6-cis-octadécènoïque (18:1n-12), peut être utilisé à l'état natif, par exemple. Cependant, les esters de l'acide pétrosélinique sont également potentiellement des activateurs de la β-oxydation peroxisomale. En effet, d'autres acides gras activateurs de la β-oxydation peroxisomale, comme l'acide palmitique ou l'acide oléique, à l'état natifs ou estérifiés, sont reconnus pour être des activateurs de la β-oxydation péroxisomale (Schmidt *et al*., Lipids, 31, 1115-1124, 1996). L'acide pétrosélinique peut ainsi être estérifié avec le glycérol (mono-, di- ou tri-acyl), un alcool comme les alcools méthylique et éthylique, un sucre, un tocophérol, un tocotriénol, un stérol ou encore un alcool gras, par exemple.

La composition peut ainsi comprendre une huile d'une ombellifère riche en acide pétrosélinique. On entend par "huile riche en acide pétrosélinique", une huile contenant au moins 40% d'acide pétrosélinique.

Les ombellifères sont des plantes dont les fleurs sont disposées en ombelles. Les espèces de préférence utilisées dans l'invention sont le coriandre, le cerfeuil, la carotte, le céleri, le cumin et l'aneth. L'huile d'ombellifère utilisée selon l'invention est extraite de la graine de cette ombellifère, par exemple par broyage ou pressage, puis raffinage. L'huile d'ombellifère a une teneur en acide pétrosélinique qui varie selon la graine d'ombellifère d'où elle est extraite. Pour une même ombellifère, la teneur en acide pétrosélinique varie aussi selon le pays d'origine de l'ombellifère et selon l'extraction qui peut être plus ou moins complète. Ainsi, l'huile de graines de coriandre comporte environ de 75 à 90% d'acide pétrosélinique, l'huile de graines de cumin en comporte environ 55 à 65%, l'huile de graines de carotte environ de 55 à 65%, l'huile de graines de persil environ de 40 à 50%, l'huile de graines de fenouil environ de 55 à 65%, l'huile de graine de cerfeuil environ de 50 à 70%, l'huile de graines de céleri environ de 45 à 60%, l'huile de graines d'aneth environ de 75 à 85%, ces pourcentages étant donnés par rapport au poids total de la composition.

L'huile peut être utilisée dans la composition selon l'invention à une concentration allant de 0,01 à 50% en poids par rapport au poids total de la composition, par exemple, et de préférence de 0,2 à 20%.

Il faut remarquer que selon les formes galéniques normalement utilisées pour une application topique ou orale, c'est à dire selon les formes alimentaire, cosmétique et/ou pharmaceutique, la quantité d'acide pétrosélinique suffisante et nécessaire pour observer un effet anti-inflammatoire ou modulateur du métabolisme des lipides peut considérablement varier. A cet effet, l'invention a également pour objet l'utilisation de l'acide pétrosélinique dans une quantité suffisante pour le traitement ou la prévention des inflammations et/ou pour la modulation du métabolisme des lipides des tissus superficiels.

Dans le domaine de la cosmétique, humaine ou animale, l'invention a encore pour objet l'utilisation de l'acide pétrosélinique, notamment contenu dans une huile d'ombellifère, pour préparer une forme galénique normalement utilisée pour une application topique ou d'hygiène buccale, et notamment sous forme de solutions huileuses, alcooliques ou hydroalcooliques, de gels, d'émulsions eau-dans-huile ou huile-dans-eau, ayant l'aspect d'une crème ou d'un gel, éventuellement aptes à mousser, sous forme d'aérosol, ou encore sous forme de dispersions vésiculaires contenant des lipides ioniques et/ou non ioniques. Ces formes galéniques sont, de toute façon, préparées selon les méthodes usuelles des domaines cosmétiques considérés. Cette composition peut constituer notamment une composition de nettoyage, de protection, de traitement ou de soin pour le cuir chevelu, pour le visage, pour le cou, pour les mains ou pour le corps (par exemple sous forme de crèmes de jour, crèmes de nuit, crèmes démaquillantes, crèmes ou huiles solaires, huiles pour le corps ou le visage, laits de nettoyage, laits de démaquillage, des laits corporels), une composition de maquillage (par exemple fond de teint), une composition de bronzage artificiel, une composition pour le bain, une composition shampooing (traitement du cuir chevelu), ou une composition pour l'hygiène buccale (bain de bouche, dentifrice, pâte à mastiquer), par exemple.

Dans le domaine de la pharmacie, humaine ou animale, notamment de la dermatologie, de l'oto-rhino-laryngologie de l'ophtalmologie, de la gastro-entérologie et de la gynécologie, cette composition peut être une capsule, une gélule, une émulsion, une pommade, une composition infectable sous la peau, un onguent, un sirop, un diffuseur, un collyre, un shampooing ou un bain de bouche, renfermant de l'acide pétrosélinique, notamment contenu dans une huile d'ombellifère, destiné pour le traitement ou la prophylaxie de la peau, de l'oeil ou des muqueuses, par exemple. Les différentes formes galéniques possibles sont, de toute façon, préparées selon les méthodes usuelles des domaines pharmaceutiques considérés.

Enfin dans le domaine de l'alimentation, humaine ou animale, cette composition peut être toute composition ingestible, liquide ou solide, renfermant de l'acide pétrosélinique, notamment contenu dans une huile d'ombellifère. Cette composition peut être une une sauce comme une sauce à salade, une huile de table, une mayonnaise, une crème glacée, une composition de pâtisserie, une pâte de fourrage ou à tartiner, par exemple.

La présente invention est décrite plus en détail par les exemples présentés ci-après. Il va de soi, toutefois, que ces exemples sont donnés à titre d'illustration de l'objet de l'invention dont ils ne constituent en aucune manière une limitation. Les pourcentages sont donnés en poids sauf indication contraire.

### Exemple 1 Activation des kératinocytes

1) Effet anti-inflammatoire: les kératinocytes expriment naturellement des protéines et des enzymes impliquées dans les réponses inflammatoires après traitement avec des agents pro-inflammatoires, comme des esters de phorbol. Parmi les protéines et les enzymes ainsi produites, on peut ainsi compter la superoxyde-dismutase (SOD), et la collagénase de type I et le facteur de nécrose tumorale alpha (TNFα), par exemple.

Dans les essais qui suivent, des kératinocytes humains primaires ou immortalisées en culture sont soumis *in-vitr*o à un traitement pro-inflammatoire avec du 12-*O*-tetradecanoylphorbol acétate (TPA). Les lignées immortalisées de kératinocytes DK2, DK7, FK2 décrites dans PCT/EP96/05812 (Société des Produits Nestlé) ont été à cette fin utilisées. Les lignée FK2 et DK2 ont d'ailleurs été déposées, le 5 octobre 1995, sous le traité de Budapest, à la DSM (Deutsche Sammlung von Mikroorganismen und Zellkulturen GmbH, Mascheroder Weg 1b, D-38124 Braunschweig, Allemagne) et se sont vues attribuer respectivement les numéros de dépôt DSM ACC2240 et DSM ACC2238. La lignée immortalisée de kératinocytes HaCaT décrite par Boukamp *et al*. a également été utilisée (J. Cell. Biol., 106, 761-771, 1988).

On ensemence une boite de Petri (6 cm ⌀) contenant 4 ml de milieu NR-2 (Biofluids), décrit dans PCT/EP96/05812, avec les lignées primaires ou immortalisées susmentionnées. Arrivé à confluence, on remplace le milieu par du milieu frais NR-2 riche en calcium (CaCl2, 1,5 mM). On incube pendant 2 jours, et on remplace à nouveau le milieu par du milieu frais, et on incube à nouveau pendant 2 jours. On nourrit ensuite les cellules pendant 2 jours avec du milieu NR-2 riche en calcium (CaCl2, 1,5 mM) contenant 1 mg/ml de BSA et un acide gras donné (ac. oléique ou pétrosélinique) ou une quantité équivalente du solvant utilisé pour les acides gras (éthanol).

Pendant les dernières 24h, on ajoute au milieu 10ng/ml de TPA ou une quantité équivalente d'éthanol. On collecte ensuite les surnageants, et on mesure par ELISA la teneur en TNFα ainsi produit. Les anticorps spécifiques contre le TNFα sont disponibles dans le commerce (BioSource Inter., US, n° catalogue KHC0012 et KHC3013).

Les résultats, dont une partie est présentée au tableau 1 ci-dessous, montrent que l'incubation des kératinocytes humains avec de l'acide pétrosélinique conduit à une inhibition de la production de TNFα par les cellules soumises à un agent irritant. Cette inhibition est spécifique puisque l'acide oléique (témoin) n'a aucun effet d'inhibition. De plus, cette inhibition est dépendante de la dose appliquée en acide pétrosélinique.

**Tableau 1**

| Inflammations des kératinocytes DK2 | | |
|---|---|---|
| Traitement | pg/ml TNFα | % par rapport à la valeur contrôle |
| TPA + EtOH (Témoin) | 41.0 | 100 |
| TPA + 25µM d'acide oléique | 39.8 | 97.1 |
| TPA + 50µM d'acide oléique | 36.3 | 88.5 |
| TPA + 100µM d'acide oléique | 45.6 | 111.2 |
| TPA + 200µM d'acide oléique | 37.8 | 92.2 |
| TPA + 25µM d'acide pétrosélinique | 35.6 | 86.8 |
| TPA + 50µM d'acide pétrosélinique | 23.1 | 56.3 |
| TPA + 100µM d'acide pétrosélinique | 20.0 | 48.8 |
| TPA + 200µM d'acide pétrosélinique | 12.9 | 31.4 |
| (Le témoin sans TPA: 0,7 pg de TNFα/ml) | | |

2) Catabolisme de l'acide pétrosélinique: on détermine le profile en acides gras des kératinocytes primaires ou immortalisées.

Pour cela, on incube les cellules pendant 2 fois 2 jours (renouvellement du milieu après les 2 premiers jours) dans du milieu NR-2 riche en calcium (CaCl2, 1,5 mM) contenant 1 mg/ml de BSA et un acide gras donné (100µM d'ac. oléique 18:1n-9; 100µM d'ac. pétrosélinique 18:1n-12) ou une quantité équivalente de solvant utilisé pour les acides gras (éthanol: témoin).

On lave ensuite les cellules 1 fois dans un tampon HBSS (Hanks Balanced Salt Solution, Biofluids n°325) contenant 0,1% de BSA, 2 fois dans le tampon HBSS. On détache ensuite les cellules dans 1 ml HBSS, on les centrifuge, et on extrait les cellules dans un mélange d'héxane et d'isopropanol 2/1 (v/v) contenant 0,01% de 2,6-di-tert-butyl-p-crésol. On sépare ensuite les phospholipides par chromatographie sur couche mince (silica gel 60) avec comme éluant un mélange de chloroforme et d'acétone 96/4 (v/v). Les acides gras des phospholipides sont ensuite estérifiés par chauffage dans une solution contenant 10% de BF3-méthanol. Les esters méthyliques sont alors séparés, quantifiés par chromatographie gazeuse-liquide avec une détection par flamme ionisée. Les esters sont identifiés par comparaison avec des témoins appropriés. L'identification des acides gras est aussi confirmée par chromatographie gazeuse

couplée à la spectrométrie de masse, après dérivativation avec le 4,4-diméthyl-oxazoline, comme décrit par Fay *et al*., J. Chromat., 541, 89-98, 1991.

Les résultats, dont une partie sont présentés aux tableaux 2 et 3 ci-dessous, montrent que l'acide pétrosélinique est incorporé dans la fraction phospholipidique des cellules. Il est aussi catabolisé par β-oxydation (probablement par les péroxisomes) du fait de la présence en quantité importante de l'acide delta-4-cis-hexadécénoique (16:1n-12). Ce métabolite et son homologue, le 16:1n-9 dérivé de la β-oxydation de l'ac. oléique, ne sont pas détectés lorsque les cellules sont incubées en présence d'acide oléique.

**Tableau 2**

| | Cellules primaires | | | DK7 | | |
|---|---|---|---|---|---|---|
| Acides gras | témoin | + 100 µM 18:1n-9 | + 100 µM 18:1n-12 | témoin | + 100 µM 18:1n-9 | + 100 µM 18:1n-12 |
| 14:0 | 3,86 | 2,20 | 1,31 | 2,59 | 2,24 | 1,40 |
| 16:0 | 17,62 | 11,09 | 5,94 | 15,64 | 11,94 | 5,26 |
| 16:1n-10 | 4,57 | 4,42 | nd | 1,61 | 3,06 | nd |
| 16:1n-12 | 0,00 | 0,00 | **22,07*** | 0,00 | 0,00 | **22,69*** |
| 16:1n-7 | 13,11 | 4,67 | 4,71 | 15,38 | 6,00 | 7,15 |
| 18:0 | 10,65 | 7,95 | 4,88 | 7,06 | 4,76 | 3,22 |
| 18:1n-9 | 30,39 | 55,86 | nd | 27,63 | 51,84 | nd |
| 18:1n-12 | 0,00 | 0,00 | **49,88*** | 0,00 | 0,00 | **44,23**** |
| 18:1n-7 | 12,56 | 5,81 | 5,96 | 23,40 | 11,77 | 11,90 |
| 18:2n-6 | 1,15 | 0,61 | 0,59 | 0,85 | 0,24 | 0,34 |
| 20:0 | 0,26 | 0,05 | 0,00 | 0,26 | 0,10 | 0,07 |
| 20:1n-9 | 0,54 | 1,20 | 0,15 | 0,62 | 1,96 | 0,19 |
| 20:3n-9 | 1,54 | 1,81 | 1,33 | 1,43 | 1,49 | 1,28 |
| 20:3n-6 | 0,46 | 0,34 | 0,28 | 0,26 | 0,14 | 0,15 |
| 20:4n-6 | 1,27 | 1,42 | 1,56 | 0,87 | 0,67 | 0,87 |
| 20:5n-3 | 0,14 | 0,06 | 0,00 | 0,25 | 0,00 | 0,03 |
| 22:0 | 0,31 | 0,30 | 0,35 | 0,28 | 0,15 | 0,13 |
| 22:1n-9 | 0,33 | 0,30 | 0,00 | 0,26 | 0,65 | 0,03 |
| 22:2n-6 | 0,62 | 0,68 | 0,52 | 0,81 | 0,76 | 0,43 |
| 22:4n-6 | 0,00 | 0,08 | 0,00 | 0,00 | 0,06 | 0,04 |
| 22:5n-3 | 0,00 | 0,09 | 0,00 | 0,00 | 0,00 | 0,05 |
| 22:6n-3 | 0,00 | 0,00 | 0,00 | 0,00 | 0,00 | 0,00 |
| 24:1n-9 | 0,62 | 1,04 | 0,35 | 0,81 | 2,17 | 0,55 |

| | | | | | | |
|---|---|---|---|---|---|---|
| * peut contenir des traces de 16:1n-10 | | | | | | |
| ** peut contenir des traces de 18:1n-9 | | | | | | |
| nd : non détectable | | | | | | |

**Tableau 3**

| | DK2 | | | FK2 | | |
|---|---|---|---|---|---|---|
| Acides gras | témoin | + 100 µM 18:1n-9 | + 100 µM 18:1n-12 | témoin | + 100 µM 18:1n-9 | + 100 µM 18:1n-12 |
| 14:0 | 1,73 | 2,53 | 2,23 | 6,85 | 3,46 | 3,09 |
| 16:0 | 17,96 | 16,42 | 12,98 | 15,04 | 14,23 | 11,51 |
| 16:1n-10 | 3,51 | 1,68 | nd | 3,71 | 3,14 | nd |
| 16:1n-12 | 0,00 | 0,00 | **11,79*** | 0,00 | 0,00 | **12,12*** |
| 16:1n-7 | 9,42 | 5,64 | 7,49 | 16,56 | 6,77 | 7,44 |
| 18:0 | 9,84 | 8,18 | 6,30 | 4,23 | 4,53 | 4,26 |
| 18:1n-9 | 36,48 | 49,33 | nd | 26,53 | 43,26 | nd |
| 18:1n-12 | 0,00 | 0,00 | **42,01**** | 0,00 | 0,00 | **38,59**** |
| 18:1n-7 | 14,30 | 7,98 | 11,55 | 20,94 | 16,03 | 16,81 |
| 18:2n-6 | 0,25 | 0,22 | 0,27 | 0,30 | 0,27 | 0,37 |
| 20:0 | 0,28 | 0,16 | 0,00 | 0,00 | 0,14 | 0,10 |
| 20:1n-9 | 0,96 | 2,09 | 0,42 | 0,63 | 1,78 | 0,50 |
| 20:3n-9 | 2,50 | 2,72 | 2,61 | 2,17 | 2,17 | 2,17 |
| 20:3n-6 | 0,03 | 0,07 | 0,08 | 0,08 | 0,05 | 0,06 |
| 20:4n-6 | 0,29 | 0,27 | 0,33 | 1,02 | 0,55 | 0,58 |
| 20:5n-3 | 0,02 | 0,02 | 0,05 | 0,08 | 0,05 | 0,07 |
| 22:0 | 0,39 | 0,22 | 0,20 | 0,11 | 0,20 | 0,17 |
| 22:1n-9 | 0,20 | 0,36 | 0,11 | 0,31 | 0,64 | 0,20 |
| 22:2n-6 | 0,73 | 1,04 | 0,87 | 0,86 | 1,20 | 1,03 |
| 22:4n-6 | 0,00 | 0,00 | 0,05 | 0,00 | 0,02 | 0,01 |
| 22:5n-3 | 0,02 | 0,02 | 0,00 | 0,01 | 0,00 | 0,02 |
| 22:6n-3 | 0,00 | 0,00 | 0,00 | 0,00 | 0,00 | 0,00 |
| 24:1n-9 | 0,91 | 1,06 | 0,67 | 0,56 | 1,51 | 0,90 |

| | | | | | | |
|---|---|---|---|---|---|---|
| * peut contenir des traces de 16:1n-10 | | | | | | |
| ** peut contenir des traces de 18:1n-9 | | | | | | |
| nd : non détectable | | | | | | |

### Example 2 Activation des cellules du colon

Les cellules épithéliales du colon humain expriment naturellement des protéines et des enzymes impliquées dans les réponses inflammatoires après traitement avec des agents pro-inflammatoires, comme des esters de phorbol. Parmi les protéines et les enzymes ainsi produites, on peut ainsi compter la superoxyde-dismutase (SOD) et le facteur de nécrose tumorale alpha (TNFα), par exemple.

Dans les essais qui suivent, des cellules épithéliales du colon humain en culture, primaires et immortalisées, sont soumis *in-vitro* à un traitement inflammatoire avec du TPA. La lignée immortalisée est celle décrite dans EP96201064.1 (Société des Produits Nestlé) qui a été déposée, sous le traité de Budapest, auprès de la Deutsche Sammlung von Mikroorganismen und Zellkulturen, Mascheroder Weg 1b, D-38124 Braunschweig, Allemagne, le 16 avril 1996, où elle a reçu le numéro de dépôt DSM ACC2258.

Comme décrit à l'exemple 1, on détermine l'effet de l'acide pétrosélinique sur la production de TNFα par les cellules du colon, lorsque celles-ci sont soumises à un traitement irritant. Le milieu de culture B50 (Biofluids), décrit dans EP96201064.1, est à cette fin utilisé. Les résultats des essais sont comparables à ceux présentés à l'exemple 1.

### Example 3 Activation des cellules de la comée

Les cellules épithéliales de la cornée humaine expriment naturellement des protéines et des enzymes impliquées dans les réponses inflammatoires après traitement avec des agents pro-inflammatoires, comme des esters de phorbol. Parmi les marqueurs d'une réaction inflammatoire, on peut ainsi compter la collagénase I (marqueur facilement détectable: Bazan *et al*., Proc. Natl. Acad. Sci. USA, 90, 8678-8682, 1993), le c-fos (voir Bazan *et al*.), les intermédiaires arachidoniques 12-HETE et 12-HETrE (Conners *et al*., Inves. Ophth. & Visu. Sci., 36, 828-840, 1995) et un nouveau profile en cytokines et en facteurs de croissance, par exemple.

Dans les essais qui suivent, des cellules épithéliales de la comée humaine en culture, primaires ou immortalisées, sont soumises *in-vitro* à un traitement inflammatoire avec du TPA. La lignée immortalisée est celle décrite dans EP96203707.3 (Société des Produits Nestlé) qui a été déposée, sous le traité de Budapest, auprès de la Collection Nationale de Cultures de Microorganismes, Institut Pasteur, 25 rue du Docteur Roux, 75724 Paris, le 22 octobre 1996, où elle a reçu le numéro de dépôt CNCM I-1777. Le milieu de culture utilisé est le milieu KGM-1 (= milieu KBM de Clonetics, US, comprenant en outre 0,15 mM de CaCl₂, 30 µg/ml d'extrait de glande pituitaire bovine, 0,5µg/ml d'hydrocortisone, 5 µg/ml d'insuline, 10 µg/ml de transférine, 10 ng/ml de facteur de croissance épidermal murin (EGF), 10000 U/ml de pénicilline et 10000 U/ml de streptomycine).

Comme décrit à l'exemple 1, on détermine l'effet de l'acide pétrosélinique sur la production de collagénase I par les cellules de la comée, lorsque celles-ci sont soumises à un traitement irritant. Pour cela, on peut détecter l'expression de la collagénase par PCR, par Western-Blot ou par Elisa.

Pour détecter la collagénase I par PCR, on extrait l'ARN des cellules sensibilisées, et on détecte la présence d'ARNm de collagénase I par PCR avec une amorce dérivée de la séquence d'ADN de la collagénase I (Genebank: n°X05231).

Pour détecter la collagénase I par Western-Blot, on récupère le milieu de culture sensibilisé, on le concentre avec un filtre Amicon30®, on sépare les protéines par électrophorèse sur un gel de polyacrylamide SDS-Page, on les transferère par Western-blot sur un filtre, on soumet le filtre à un premier anticorps anti-collagénase I (Cortex Biochem, US, n° 60338P), à un deuxième anticorps couplé à une péroxydase (Pierce, US, n° 31400), puis au substrat de la péroxydase.

Pour détecter la collagénase I par Elisa, il suffit d'utiliser le kit hMMP-1 d'Amersham (catalogue, rpn 2610), en suivant les recommandations du fournisseur.

Les résultats des essais sont comparables à ceux présentés à l'exemple 1.

### Example 4 Lait corporel (émulsion huile-dans-eau)

### Phase huileuse :

| | |
|---|---|
| Glyceryl stearate/PEG-100 stearate (Arlacel 165 vendu par la société ICI) (émulsionnant) | 1 % |
| Polysorbate 60 (émulsionnant) | 0.8 % |
| Polyisobutène hydrogéné | 2 % |
| Acide stéarique | 1 % |
| Huile de graines de coriandre | 8 % |

### Phase aqueuse :

| | |
|---|---|
| Glycérine Carbomer (carbopol 941 vendu par la | 3 % |
| société Goodrich) (gélifiant) | 0.3 % |
| Triéthanolamine (neutralisant) | 0.3 % |
| Conservateur | 0.3 % |
| Eau | qsp 100% |

On prépare l'émulsion en incorporant la phase huileuse dans la phase aqueuse sous agitation. On obtient un lait corporel qui assure une bonne protection de la peau contre les inflammations.

### Exemple 5 Fluide de soin (émulsion huile-dans-eau)

### Phase huileuse :

| | |
|---|---|
| Méthylglucose sesquistéarate (émulsionnant) | 2 % |
| Cyclométhicone | 13 % |
| Huile de granies de corandre | 2 % |
| Parfum | 0,2 % |
| PEG 20 méthylglucose sesquistéarate (émulsonnant) | 2 % |

### Phase aqueuse :

| | |
|---|---|
| Gommes de xanthane (gélifiant) | 0,2 % |
| Polysacrylamide/isoparaffine C13-C14/leureth-7 (Sepigel 305 vendu par la société Seppic) (gélifiant) | 0,8 % |
| Conservateur | 0,3 % |
| Eau | qsp 100% |

On prépare l'émulsion comme dans l'exemple 4. On obtient un fluide blanc, qui assure une bonne protection à la peau contre les inflammations.

### Exemple 6 Crème de soin (émulsion eau-dans-huile)

### Phase huileuse :

| | |
|---|---|
| Polyglyceryl-4 Isostéarate/Cetyl dimethicone copolyol/Hexyl laurate (Abil WE 09 vendu par la société Goldschmidt) (émulsionnant) | 4 % |
| Isohexadécane | 5 % |
| Huile de graines de coriandre | 10 % |
| Cyclométhicone | 3,5 % |
| Acide n-octanoyl-5-salicylique | 1 % |
| Parfum | 0,15 |

### Phase aqueuse :

| | |
|---|---|
| Glycérine | 10 % |
| Gomme de cellulose | 0,5 % |
| Sulfate de magnésium | 0,65 % |
| Conservateur | 0,3 % |
| Eau | qsp 100 % |

Pour préparer l'émulsion, on chauffe les constituants de la phase A à 80°C jusqu'à dissolution complète des différents constituants, puis on la refroidit à 65°C. On chauffe la phase B à 65°C et on la verse dans la phase A sous agitation, puis on refroidit le mélange. On obtient une crème blanche, lisse, nourrissante, qui assure une bonne protection de la peau contre les inflammations.

## Revendications

1. Utilisation de l'acide pétrosélinique, natif ou estérifié, pour la préparation d'une composition destinée à activer la β-oxydation péroxisomale des acides gras dans les tissus superficiels d'un mammifère de sorte à pouvoir traiter ou prévenir les inflammations et/ou moduler le métabolisme des lipides dans les tissus superficiels.

2. Utilisation selon la revendication 1, caractérisée en ce que la composition est destinée à moduler les processus cellulaires faisant intervenir les cytokines.

3. Utilisation selon la revendication 2, caractérisée en ce que les tissus superficiels sont les cellules d'un mammifère constituant la peau, le cuir chevelu, l'oeil, ou les muqueuses orale, nasale, buccale ou vaginale.

4. Utilisation selon la revendication 1, caractérisée en ce que la composition est destinée à une application topique ou orale.

5. Utilisation selon la revendication 1, caractérisée en ce que la composition comprend une huile d'une ombéllifère qui est riche en acide pétrosélinique.

6. Utilisation selon la revendication 5, caractérisée en ce que l'huile comprend au moins 40% d'acide pétrosélinique.

7. Utilisation selon la revendication 5, caractérisée en ce que la quantité d'huile va de 0,01 à 50% en poids par rapport au poids total de la composition.

8. Utilisation selon l'une quelconque des revendications 1 à 7, caractérisée en ce que l'acide pétrosélinique est présent dans une quantité suffisante pour le traitement ou la prévention des inflammations et/ou pour la modulation du métabolisme des lipides des tissus superficiels.

9. Utilisation selon l'une quelconque des revendications 1 à 7, caractérisée en ce la composition est une composition cosmétique, pharmaceutique et/ou alimentaire.
